# EUROPEAN PATENT APPLICATION

(11) **EP 2 246 798 A1**
(43) Date of publication of application: **03.11.2010**
(21) Application number: 09173374.1
(22) Date of filing: 19.10.2009
(51) Int. Cl.: G06F 19/00

(54) **Method and system for managing and displaying medical data**

(30) Priority: 30.04.2009 EP 09159227
(71) Applicant: TomTec Imaging Systems GmbH, 85716 Unterschleissheim (DE); Amid s.r.l., 67039 Sulmona (AQ) (IT)
(72) Inventor: Tonti, Giovanni, 67039 Sulmona (IT); Pedrizzetti, Gianni, 50047 Prato (IT); Baumann, Rolf, 88239 Wangen (DE); Mumm, Bernhard, 82291 Mammendorf (DE); Waldinger, Johannes, 85521 Ottobrunn (DE)
(74) Representative: Müller, Frank Peter

(57) **Abstract**

The invention relates to a medical data management method and system wherein medical data (22c) and identification data (22b) associated with the medical data (22c) of said patient (6) is received, said medical data (22c) and said identification data (22b) is stored as a medical file (22) in a storage system (2), wherein at least parts of said medical files (22) are modified for generating at least one modified file (22') in response to a retrieval request from an unauthorized recipient (Ni), wherein such modification depends on an authorization code (42) associated with said unauthorized recipient (Ni), and wherein said at least one modified file (22') is supplied to said unauthorized recipient (Ni) or the unmodified medical file (22) is supplied to an authorized recipient (6, 7). The invention enables a worldwide medical database for research and global health interests, by at the same time safeguarding the legitimate interests of the patient.

## Description

The present invention relates to a method for managing and displaying distributed digital medical data, especially medical images in accordance with claims 1, a system for managing and displaying distributed digital medical data in dependency of an approval routine in accordance with claim 9 and a software module for performing the claimed method in accordance with claim 15.

Existing medical information management methods and systems are typically categorized by the types of information they handle. For example: picture archiving and communication systems (PACS) handle the storage and retrieval of digital images, radiology information systems (RIS) handle patient demographics, exam scheduling, and storage and retrieval of radiology reports, laboratory information system (LIS) are responsible for the storage and retrieval of lab results, hospital information systems (HIS) handle patient demographics, payer information, scheduling and coordination of care across the hospital, computerized patient order entry (CPOE) systems take instructions from physicians as to patient care and distribute tasks to other caregivers, and electronic medical record (EMR) systems handle the digital acquisition and retrieval of the complete patient record often relying upon a storage system termed a clinical data repository (CDR).

A topic of great importance to the medical community is the means by which these existing systems can be integrated within and across given healthcare enterprises. Internet web technologies have been applied to provide standard user interfaces by which patient information is shared between affiliated medical institutions through local area networks (LANs) or wide area networks (WANs). These solutions, however, are generally restricted to the sharing of digital data between affiliated entities such as hospitals and clinics within a single IT system. This is mainly due to the fact that medical data remains largely analogical in nature, that is, paper- and film-based. When patient information is contained in digital form, the formats are typically without accepted or implemented standard representations. Some communications standards, however, do exist. HL7 or Dicom is a standard for electronic data interchange in healthcare environments.

The standard (originally developed in 1987 by a group of large healthcare providers who met at the University of Pennsylvania) at first emphasized point-to-point transmission of patient-oriented admission-discharge-transfer (ADT) order, and results information in inpatient environments. Today, HL7 prescribes formats for the interchange of information concerning all aspects of the healthcare enterprise, including billing, clinical pathways, care guidelines, referrals, and information about practitioners.

One general area of medical practice overcoming the obstacles to standardized digital data sharing is that of radiology, or diagnostic imaging, where a great deal of patient information is either inherently digital (e.g. magnetic resonance imaging (MR), computed tomography (CT), positron emission tomography (PET), etc.) or acquired digitally (computed radiography, digital radiography). Over the last years, hospitals have not only adopted digital radiological systems in large quantity, but are also implementing PACS for storing, interpreting and distributing images in their original digital form. The field of radiology is also a leader with respect to digital data standards, having created and adopted the Digital Imaging and Communication in Medicine or DICOM standard, which is universally accepted and implemented around the world (see 2001 Digital Imaging and Communication in Medicine (DICOM). NEMA Publications PS 3.1 PS 3.12. Rosslyn, Va.: The National Electrical Manufacturers Association (see http://medical.nema.org).

A picture archiving and communication system (PACS) consists of image and data acquisition, storage, and display subsystems integrated by various digital networks. It can be as simple as a modality connected to a display workstation with small image database, or as complex as a total hospital image management system. Now, most clinical PACS developed as open architecture systems are following the DICOM standard in image communication, image format and image management. The image distribution and display inside radiology departments or hospitals are mostly using DICOM services, e.g. Storage, Query/Retrieval, Printing, etc., and these standardized services greatly and efficiently improve the interpretabilities among different manufactures' PACS components. Image display workstations as the major interfaces for users accessing PACS images are the last components in the PACS data flow. They can be loosely categorized into different types based on their applications, such as, diagnostic, review, analysis, and interactive teaching, as well as desktop workstations for surgical simulation, radiation therapy planning and other applications. The diagnostic workstations are used by the radiologists for making primary diagnosis, they are mostly equipped with dedicated hardware such as multi-portrait mode high resolution monitors, and powerful computer with large memory and fast CPU and GPU, as well as special designed display software to handle multi-modalities image communication, examination and image navigation, image processing and manipulation and work flow management with patient or study related information.

The evolution of low cost and powerful personal computers and Internet technology enable multimedia information to be ubiquitous. In recent years, Web-based PACS has thus become mainstream for the enterprise-wide distribution of medical images. Compared with the legacy PACS, which is developed on the basis of a dedicated high-speed network infrastructure, Web-based PACS uses relatively low speed intranet or Internet TCP/IP (Transmission Control Protocol/ Internet Protocol), which takes advantage of existing low cost communication infrastructures. It also includes simple personal computers, and Web browsers with Internet-related technologies. On the other hand, traditional PACS use powerful workstations with high-resolution monitors, and proprietary software. Thus, Web-based PACS can solve the enterprise-wide image distribution issue efficiently and economically.

The first Web-based PACS which appeared on the market were pure link interfaces between a traditional PACS database and a Web server which creates web pages "on the fly" upon request. An example of such a system is General Electric's "Web-Link" component of their workstation-based PACS.

An evolution of this system is disclosed in US patent n. 6,934,698 which relates to a medical management system to allow any conventional Internet browser to function as a medical workstation. The core of the therein-described invention is a Web-compatible database located on a http Server which can be interrogated from any location on earth. The images are pulled from commercial scanners in response to a user request, processed without loss of diagnostic information, adjusted with respect to brightness and contrast and posted on Internet web pages for viewing. An e-mail server notifies the requester that the images have been posted and can thus be downloaded after proper authentication.

An alternative to a centralized web-compatible database is disclosed in US patent n. 7,234,064 which relates to a method and apparatus for creating a secure, centrally-mediated, peer-to-peer network of healthcare providers requiring no pre-existing affiliations or knowledge of each other. Here a central system is used for managing patient authorizations, while the image data to be fetched are located in a distributed database.

Web-based PACS, although being extremely powerful, suffer from an intrinsic limitation which is related to privacy. Medical data are in fact sensible data protected by international data privacy regulations including HIPAA in the United States and the Directives of the European Council and thus cannot be accessed by unauthorized persons.

While there have been various disclosures and proposal for methods to connect parties for the purpose of sharing digital medical information, significant obstacles still remain to communication between parties not possessing an a priori relationship and thus to the dissemination of valuable information which is the more valuable when the medical activity and medical imaging are performed with high quality standards. This valuable information would be a useful support for progressing the scientific, medical research, it would be of interest to private companies (pharmacological, medical equipments...), as well as to public agencies (social security, government budgeting...) to ensure transparency, to better monitor and control the healthcare. To cite a few examples. Notwithstanding this, the valuable information produced by every Institution is used in minimal part only. It is used primarily for regulatory archiving, then for diagnostics support and patient follow-up, sometime for internal scientific research, and internal controls. It represents mostly a cost only, not a resource.

The awareness of the potential benefits of a worldwide diffusion of medical data is growing in these years. Some services began to appear on the Internet for the sharing of anonymous medical data for informational, educational and academic purposes. WebPAX (see http://www.webpax.com), for example, is specifically intended for discussions of interesting cases, a library of images depicting various disease conditions, discussions of emerging imaging technologies and technical analyses of scanner capabilities.

These Web services provide to their customers the ability to upload, view, share, download and discuss digital medical images in the world wide web. The core is the anonymization of all files so that patient privacy is maintained. Data are anonymized upon upload to the web server by their owner and thus freely shared within the community.

These services go in the right direction for potential world wide data sharing of medical data, however the information exchanged are limited to those data uploaded and anonymized by the users. The uploaded data are not verified, and their medical effectiveness, relevance, and congruence cannot be assessed. In parallel, the additional information provided by the user who uploaded the data cannot be verified as well. These available data are thus not certified by any reliable entity or system. They are employed to navigate, get some explorative hints. They cannot be used for scientifically-based health-related progresses.

It is therefore the object of the present invention to provide an automated and universal system and a method for managing and displaying distributed digital medical data, especially medical images, that would allow to provide world-wide regulated access to relevant healthcare information documents, particularly diagnostic images and related reports, without patient authorizations, but in the full respect of patient privacy.

The present invention solves the foregoing object throughout the features of independent claims 1, 9 and 15. Preferred embodiments are described and claimed in the sublaims.

The claimed medical data management method according to the present invention comprises the steps of
- receiving medical data associated with a patient,
- receiving identification data associated with the medical data of said patient,
- storing said medical data and said identification data as a medical file in a storage system,
- modifying at least parts of said medical files and generating at least one modified file in response to a retrieval request from an unauthorized recipient, wherein such modification depends on an authorization code associated with said unauthorized recipient, and
- supplying said at least one modified file to said unauthorized recipient or supplying the unmodified medical file to an authorized recipient.

The modified file which is generated by modifying at least parts of said medical files may comprise anonymized information from such medical files or a compilation of a variety of medical information derived from several medical files in order to provide such information to an unauthorized recipient.

According to a preferred embodiment of the present invention the method comprises the further step of providing an identity provider for providing said identification data associated with said patient, with an acquisition institution and/or with other identification data, such identification data being supplied to the storage system at least partially by said acquisition institution to identify said medical data. Such identity provider can be part of the data management system of the present invention or a separate identity provider which assigns certain identification codes to acquisition institutions, patients or acquisition tools, such as medical imaging devices.

According to another preferred embodiment of the present invention acquisition data is received by the method which acquisition data is associated with the acquisition method for obtaining the medical data associated with the patient. Hence, the medical file may consist of the acquisition data, the identification data and the medical data.

The acquisition data preferably comprise a header which includes certain search elements such as the type of desease, the type of acquisition device, the type of data which is included in the medical file (such as MR, CT or ultrasonic imaging data) the format in which the data is provided (such as jpg., xml., tif....) and general anonymized patient data such as age, sex, weight, etc. The identification data preferably includes codes related to the acquisition institution (such as a hospital), the patient, the examination modalities, drugs which the patient used, equipment with which the patient has been treated, comments or diagnosis of the physicians and other delicate data which specifically relates to the patient himself.

In general, the acquisition institution generates said acquisition data which is associated with the medical data of the patient and supplies said medical file to the storage system via a parser. Such parser is prepared to modify at least parts of said medical file and generates a modified file either in response to a retrieval request from an unauthorized recipient or in accordance with a modification program wherein such modification program depends on authorization codes associated with different unauthorized recipients. That means, the parser can either modify certain medical files which are requested from unauthorized recipients depending on the authorization code of the recipient - this can be a hierarchical system where - dependent on the authorization code of the recipient - the medical file is more or less modified - or in accordance with a modification program which is run by the parser if there is time and capacity for such modification routine wherein the modification program modifies the medical files in accordance with any possible authorization codes which are provided by the identity provider.

If the identity provider for example provides three different levels of authorization codes (such as full access, anonymized access and statistical access) the parser could modify the medical files in a first step so that all medical files are anonymized so that such anonymized file can immediately be retrieved by an unauthorized recipient once the unauthorized recipient sends such authorization code and the parser could then modify in a second step the original files or the modified files by calculating data of the medical files for providing various statistical files which are then supplied as "modified files" to unauthorized recipients which send such "statistical authorization codes".

According to a preferred embodiment of the present invention said identification data comprises an exam retrieval code, which comprises an institution code with information related to the acquisition institution, a patient code with information related to the patient and/or an exam identification code with information related to the examination modalities. Said authorization code is preferably compared with said identification data of the requested medical file in order to determine those parts of the medical file which have to be modified for generating said modified file.

According to a preferred embodiment the medical data is additionally stored in a modified, preferably anonymized version and the acquisition data and the modified medical data are supplied as such modified file to any unauthorized recipient without checking the authorization code.

The medical data management method according to the present invention modifies at least parts of said medical file and generates a modified file either in response to a retrieval request from an unauthorized recipient which depends on its authorization code associated with different unauthorized recipients and/or in response to a modification program. Such modification program generates at least one modified file by using various modalities such as filtering, data mining, segmenting, classifying or standardizing, thereby generating so-called "2^{nd} level data". That is explained as follows:
Upon storage of the original medical file (containing the medical data and identification data) the present invention uses such medical data (which partially comprises identification data, e.g. on x-ray pictures or CT-pictures which are scanned together with the name, age and sex of the patient) as so-called "first level data". Then, the medical data management method runs a modification program which produces "2^{nd} level data" or further "levelled" data wherein basically the anonymized medical data is used.

The modification program can check the medical data for consistency and can also filter or complement the original medical data. Through the combination of various medical files new data can be obtained. If the medical data consists of images it is possible to automatically enhance the quality of such images, to filter the images, to put the images into a uniform format, to calculate cross-sections of such images and/or to standardize the way these images are displayed.

With the help of such modification program it is possible to use knowledge representation systems to further calculate and condition the medical data.

Through data mining methods it is possible to standardize the medical data (which might have been anonymized in before). It is for example possible to use three-dimensional ultrasonic images of the heart of a patient and to generate standardized cross-sections such as four-chamber-view, two-chamber-view etc. These standardized cross-sections can further be used for statistical calculations. Depending on the authorization code of the unauthorized recipient it is possible to provide such second level data or such further processed statistical data (3^{rd} level data, 4^{th} level data, etc...) to such unauthorized recipient.

It is furthermore possible to use images and to filter them with regard to brightness or contrast and/or to optimize the resolution in order to prepare standardized views which can be compared easily. It is furthermore possible to pack such 2^{nd} or 3^{rd} level data with "quality identifiers" which give an indication about the quality of the data received. Hence, the use of such data can be categorized and normized.

It is furthermore possible to modify the acquisition data received from the acquisition institution by using standards in order to categorize the medical data associated with such acquisition data.

By automatically generating such 2^{nd} level data it is also possible to provide calculation services for the acquisition institution by re-submitting pre-calculated data which can be normized and/or categorized by comparison with certain standards. It is also possible to provide certain categories so that the acquisition institution is automatically informed about certain risks and/or categories in which the medical data can be categorized. That facilitates the diagnoses of such medical data. If the medical data is categorized in a certain category A and if another category B is also existent, then the medical data management method can give a certain percentage that the data received is categorized in a certain "risk group" which then can be used by the acquisition institution to prepare a more founded diagnosis. The calculation services can be performed by a separate processing unit or a processing unit which is part of the parser. The parser is usually one of the components in an interpreter or compiler, which checks for correct syntax and builds a data structure (often some kind of parse tree, abstract syntax tree or other hierarchical structure) implicit in the input medical data. In the sense of the invention, however, the parser also includes other calculation and transformation means such as processing units for analyzing the created structure, means for modifying image data, analyzers, calculation means or means for the standardization or categorization tasks.

The present invention also concerns a medical data management system comprising a gateway for receiving medical data associated with a patient and identification data associated with the medical data of said patient as well as a storage system for storing said medical data and said identification data as a medical file as well as a parser which modifies at least parts of said medical files and generates at least one modified file in response to a retrieval request from an unauthorized recipient wherein such modification depends on an authorization code associated with said unauthorized recipient. Said parser then also supplies said modified file to said unauthorized recipient or supplies the unmodified file to an authorized recipient via said gateway.

The acquisition institution and/or the recipient and the medical data management system preferably comprise a PACS and connecting means for interfacing such PACS with said storage systems to transfer medical files with medical data including one or more imaging modalities. Said medical files and/or said modified files are preferably arranged in such storage systems in a hierarchical way to provide different permissions for accessing said medical files or said modified files as a function of the aforementioned authorization code associated with the unauthorized recipient.

According to one preferred modality of the present invention, the patient himself has only a read-only permission to access his own medical files while the acquisition institution, which is the originator of such data, has full read-write access to said medical files and/or to said modified files.

The present invention also concerns a software module for such medical data management system or for such medical data management method wherein such software module has means for reading said medical files from a storage system or modifying the medical files after comparing the authorization codes with the identification codes and for supplying at least one modified file to an unauthorized recipient or supplying the unmodified medical file to an authorized recipient.

The modification of said medical files (hereinafter also named "documents" or "medical documents" or "healthcare information documents"), particularly digital medical images, can be affected once the data have been transferred to the storage system (hereinafter also named "repository" or "central database"), for example in batch mode, or "on the fly" when there's an access request from an unauthorized recipient. In the former case each document is stored in the repository both in the original and in the modified form for a quicker access while in the latter case documents are stored only in the original format and the conversion or modification is made when a request for a non-authorized access is pending, for example using memory buffers. Alternatively, each document is converted "on-the-fly", but, instead of being placed in a buffer, it is stored in the repository together with the original document for an immediate subsequent non-authorized access.

According to a preferred embodiment of the present invention, documents for non-authorized recipients (hereinafter also named "users") are modified to remove the information that are not published as shareable and rendered anonymous to eliminate any reference to individual patient thus overcoming privacy problems which hampered the widespread use of healthcare information data. This allows to provide a sort of global PACS wherein all data can be browsed and/or download by everyone from everywhere, when the authorizations rules are fulfilled, thanks to the use of the Internet. All data are in principle available, not only those uploaded by their respective owners like in the prior art systems.

The invention represents a revolutionary approach to medical data management which can contribute to an enormous progress of the scientific world. An enormous amount of data (all mankind data can be shared, in perspective) can, in fact, be accessed by research, teaching and health Institutes, but also by commercial companies, for example, for clinical trials which can thus become publicly available for an easier control, transparency, and reliability. Analysis with large number of patients can be performed in very rapid times, without the need to search and involve many new patients. Physiological hypotheses can be tested quickly; therapeutic options can be verified in very rapid times without the need of trials; data and analysis can be certified and controllable at any time. The data used in trials can be certified by a system access control, and the conclusion of scientific research can be reconstructed to improve the control of diagnostic or therapeutic conclusions reducing the occurrence of biased results.

Diagnosis and therapy can be improved. Cases, in fact, can be compared with similar ones in the entire world. Therapeutic options can be verified and rare pathologies can be shared worldwide. Each patient can access his own records from everywhere, thus avoiding unnecessary repetition of tests already done in different institution with consequent reduction of costs and exposure to radiation. Exams performed in one institution, even a remote one, can be accessed by a another institution with higher quality standards in the specific pathology for additional consulting and spread diagnostic quality worldwide.

All that thanks to a system that is able to provide for medical data, particularly medical images but not limiting to such, storage, processing and sharing between healthcare providers, physicians and researchers in the life sciences as well as single patients wanting to access their own personal records. This because all healthcare information documents can be accessed either in the original version or in a modified version or in an calculated, compiled version, which, in a preferred embodiment of the present invention, consists in a digitally edited version rendered anonymous by an anonymization process.

According to an improvement as mentioned above, different types of access to the healthcare information documents are provided depending on the identity of the recipient and the identification associated to such documents. The identification associated to the documents may also contain the identification of the sender, for example as a part of the Exam Retrieval Code including, or encoding, an identification associated to the acquisition institution (hereinafter also named "institution"), to the patient and to the examination modalities, so that it is possible to provide a hierarchical access to the documents with different permission rights. For example the institution acting as the sender of the information can access the data in read/write mode for an update of the same as it normally happens with PACS.

The system according to the invention can, in fact, be interfaced with an existing PACS through connecting means for transferring to the repository healthcare information documents produced by one or more imaging modalities, but also, in a particular advantageous configuration, the system is a PACS itself, particularly a Web-based PACS, with the repository comprising a database for storing healthcare information documents produced by one or more imaging modalities at the sender institution. The parser is preferably the interface for allowing Web access to the healthcare information documents generated by the imaging modalities of each sender and stored in the repository for a subsequent authorized or non-authorized access.

The identification data may also contain an indirect reference to the patient, such as public patient data. In this case the association of a particular document to a particular patient could be reconstructed by the sender or the acquisition institution on the basis of a local archive.

The system may comprise a plurality of senders each associated with an authorization code and an identification. The healthcare information documents provided by such senders are in this case associated to one or more identifiers capable of identifying e.g. both the patient and the acquisition institution, which is usually the sender. Both the patient and the sender are thus able to access the unmodified healthcare information documents associated to such identifiers. This access, that is what may exist in the current progress pipeline, is just the basic possibility.

Data can be arranged in a hierarchical way to provide different permissions for accessing the healthcare information documents as a function of the authorization code associated to the recipient. Particularly, the recipient identified as patient has read-only permissions to accede his own healthcare information documents. A recipient identified as the acquisition institute (as sender) has full read/write (R/W) access to such documents while recipients, other than the patient and the acquisition institution, authorized to access the healthcare information documents of a patient, have read and/or append permissions (R/O) to access such documents.

This may be the case of an institution which is not the acquisition institution, but has been authorized by the patient, for example during a follow-up, to handle his healthcare documents. This allows to provide different type of access to different institutions. For example institutions authorized to access the full record of a patient, like institutions authorized on a patient-by-patient basis for example during a follow-up exam, can accede the full data with append rights for providing, for example, comments or a new diagnostic report to be held in the patient record.

The present invention provides several authorization levels, depending on the user of the system. An example is given as follows:

| | | |
|---|---|---|
| Level 1 | Full R/W authorization | e.g. acquisition institution |
| Level 2 | Reduced R/W authorization | e.g. follow up institution |
| Level 3 | R/O authorization | e.g. patient |
| Level 4 | access to images | |
| | and measurements | e.g. laboratories, universities |
| Level 5 | only access to statistical data | e.g. WHO |
| Level 6 etc.... | | |

For the clarity of this specification, the "authorized user" is a "Level 1 - User", any "unauthorized user" is any user of level 2 and/or below. That means that the system of the present invention is capable to automatically modify the data for any unauthorized user, in the example mentioned above, for any unauthorized user of level 4 or below. The authorization levels are coded through the authorization code.

Different criteria for accessing healthcare information documents can be provided. For example it may be possible that the access to data is not completely free. For example commercial institution could gain access upon payment of some money to be shared among the sending institutions as an incentive to provide best quality data for their distribution to the community.

Healthcare information documents comprise a plurality of documents, like, but not limited to, multimodal DICOM medical images, clinical records, web-based protocols like html or xml standards. Healthcare information documents are approved as shareable when they follow a certified and recognized standard for the detection of personal information.

According to an embodiment of the present invention, modified healthcare information documents comprise digital medical images that are anonymized by clearing the pixels values containing such data on the image file. The location where personal information are placed within medical images follows precise rules in every medical imaging equipment, hence modified images are generated by recognizing such equipments to be able to know where the information to be deleted is available.

Alternatively or in combination, modified healthcare information documents comprise DICOM medical images that are anonymized by deleting or modifying one or more patient's tags in the corresponding DICOM file and clearing the possibly present image areas containing patient-related information. In DICOM images, the image areas containing information other than the acquisition are defined inside the DICOM tag and can be easily masked by replacing such area. DICOM tags also contain patient information in text form or binary form. To this extent, the identification associated to the documents preferably contains information related to the acquisition device used for performing the examination so that the pixel on the images can be cleared by using one or more masks selected according to the identification of the acquisition device.

According to another embodiment, the parser can be coupled with a relational database system that allows the retrieval of information on the basis of a series of search elements, related, for example, to the type of diseases, to the therapeutic procedures, to the available characteristics of the patients, the multimodal completeness of available images and so on. Such to speed-up the retrieval of pertinent information.

In a further embodiment, the parser can redirect the access through a series of tools that perform quantitative operation on the data. For example image quantification tools can analyze the image data and the user can access the results of the automated quantification in place of the original images. A series of data may be required in statistical terms only, such that the user may access the synthetic statistical results in place of the whole original data. Such a parser potential may redirect the data for performing intensive calculation operations, like numerical simulations, to allow access of results of computation that are typically not available within single institutions. This computation power can be granted by the unlimited resourced distributed on the web, like the cloud computing, and allows to perform advanced studies at an unprecedented level.

According to a further embodiment, different types of access are provided to the healthcare information documents depending on the identity of the recipient, as provided through the authorization code, and the identification associated to such documents, the identification associated to the documents also containing the identification of the sender.

The modified healthcare information documents (modified files) particularly comprise digital medical images that are anonymized by clearing the pixels values containing such data on the image file. To such extent, the identification associated to the documents contains information related to the acquisition device used for performing the examination. In this way it is possible to clear the pixels on the images by using different masks, the selection of the appropriate mask to be used being based on the identification of the acquisition device.

Alternatively or in combination the modified healthcare information documents comprise DICOM medical images that are anonymized by deleting or modifying one or more patient's tags in the corresponding DICOM file.

The characteristics of the invention and the advantages derived therefrom will be more apparent from the following description of non-limiting embodiments, illustrated in the annexed drawings, in which:
- Fig. 1: shows a simplified block diagram of a system according to the invention,
- Fig. 2: shows a simplified block diagram of the medical file and its identification data according to the invention,
- Fig. 3: shows a simplified block diagram of a preferred DICOM-embodiment of the invention, and
- Fig. 4: shows an exemplary list of tags related to patient data in a Dicom file.

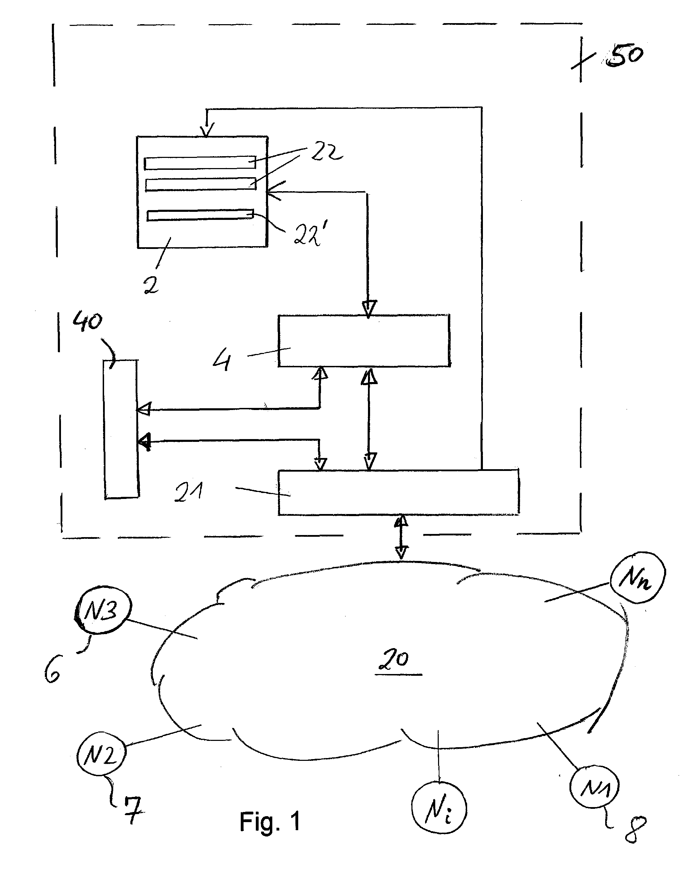

Fig. 1 shows a simplified block diagram of a system 50 according to the present invention.

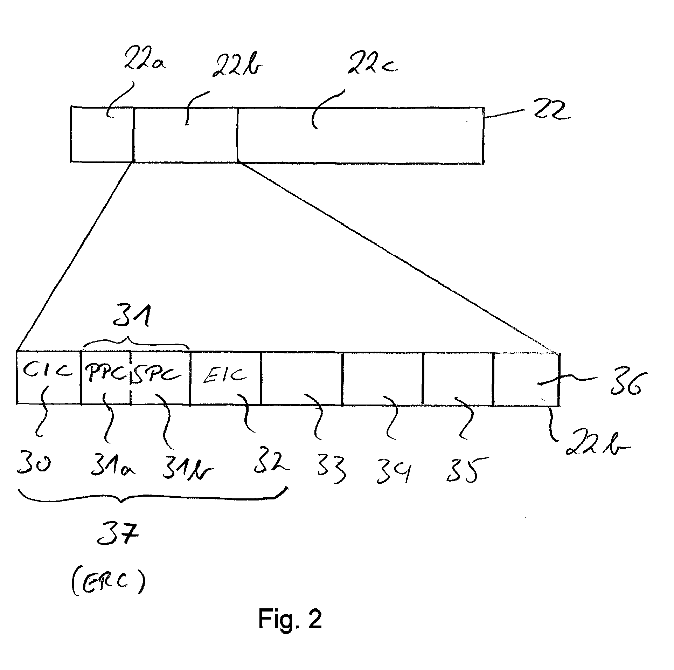

The data management system 50 comprises a gateway 21 which receives medical data 22c as identified in Fig. 2. Fig. 2 shows a simplified block diagram of the medical file 22 and its identification data 22b according to the present invention.

The medical data 22c is originated by an acquisition institution 7 which is one of a variety of users N1, N2, N3, .... Nn which are all connected to a network 20. These users N can be the acquisition institution 7, the patients 6 themselves or other institutions 8 which have an interest in accessing medical files 22 which are stored in the storage system 2 of the medical data management system 50. The gateway 21 is connected to a parser 4 which acts as a "gatekeeper" to the storage system 2. Medical data 22 can also be stored directly from the gateway 21 to the storage system 2 but not vice versa.

Upon request of an unauthorized recipient 8 or an authorized recipient 7 the parser 4 will check the submitted authorization code and will provide either a modified file 22' to an unauthorized recipient or the unmodified medical file 22 to an authorized recipient.

The system also displays an identity provider 40 which is connected to the parser 4 and the gateway 21. Each user of the system can request respective identification codes from the identity provider which supplies these identification codes in order to safeguard that all codes are universal and safeguarded. The identity provider 40 can also be separate from system 50.

As shown in Fig. 2 the medical file 22 preferably consists of acquisition data 22a, identification data 22b and medical data 22c. Often, medical data 22c also contain individual patient information such as the name, age, address and other data of the patient. The identification data 22b preferably contains an institution code 30 (CIC), patient data 31 (PPC, SPC) and an exam identification code 32 (EIC). Such individualising codes can be summarised in an exam retrieval code 37 (ERC).

Furthermore, the identification data 22b could additionally contain drug information 33, equipment information 34, physicians comments or diagnosis information 35 or other data 36. Upon request and submission of an authorization code, parser 4 will check which of the identification data and/or acquisition data and/or medical data 22c can be submitted to the recipients and parser 4 will automatically modify the medical file 22 in order to generate a modified file 22'. Such modified file could for example contain the acquisition data 22a which comprises the type of desease, the type of data included, the format in which the data is provided or other general anonymized data such as age, sex, etc. as well as drug information 33, equipment information 34, and other data 36 together with anonymized medical data 22c but not the exam retrieval code 37, no physicians comments 35 and no individualising data contained in the medical data 22c, such as the name of the patient which is often printed on medical images. Such data is removed from such medical data 22c by said parser.

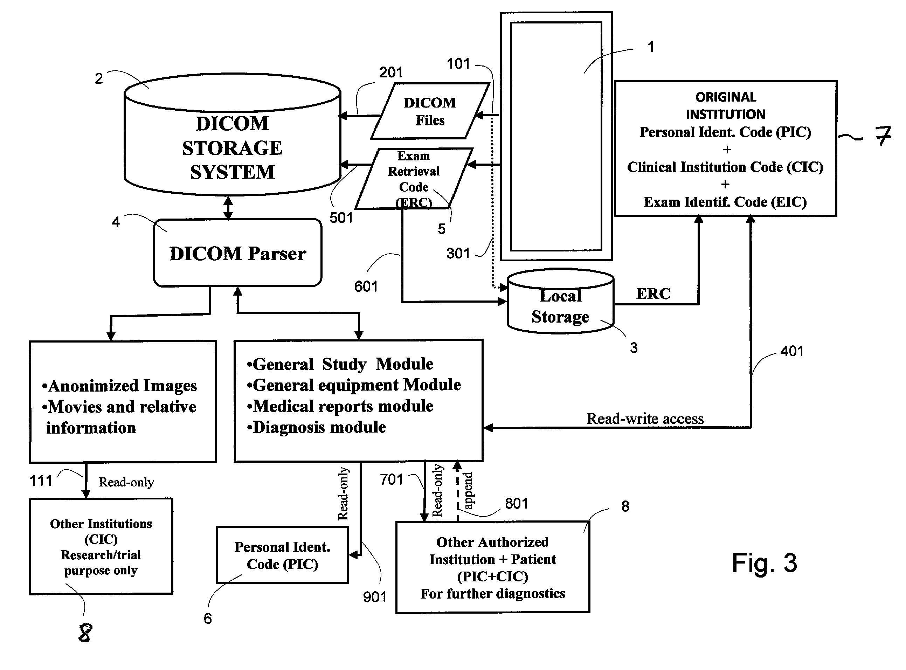

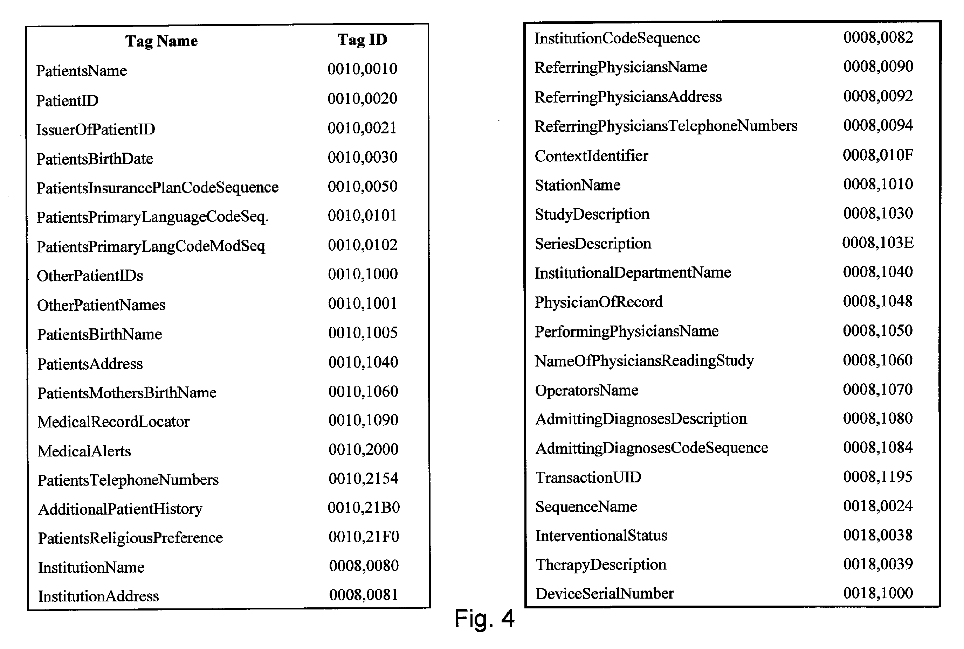

A preferred embodiment of the invention will now be described with reference to Figs. 3 and 4:
With reference to Fig. 3, the system according to the invention comprises a sender in the form of an original clinical institution (which is the acquisition institution 7) which performs multimodal exam acquisitions 1 on patients and sends the related images or sequence of images 101 as Dicom files 201 to a storage system 2 (also called "repository") indicated in the figure as Dicom storage system 2. Acquisitions 1 are performed with any known imaging modality such as TC, MRI, SPECT, PET, X-Ray or the like. The output image files can also be directed 301 to a local storage system 3 that can be a full traditional PACS or, more effectively, a database capable of just handling patient demographics and examination reports for fulfilling law obligations to keep track of the examination performed, and archive related reports, for periods depending on local regulations. As the images are transferred to the storage system 2, the local database 3 requires very little storage capability thus reducing hardware costs at the clinical institution. The images can, in fact, be downloaded in full by the original institution directly from the Dicom storage system 2 thus realizing a true Web-PACS, as it will be described hereinafter. The images stored in the Dicom storage system 2 could be directly accessed by the original institution as in conventional PACS, however, in a preferred solution as the one depicted in Fig. 3, such direct access is possible only in the upload phase of new images 201, while parsing and/or downloading is mediated 401 by the component indicated in the figure as Dicom parser 4 which subtends to the whole process for the access to data.

As shown in Fig. 1, the parser 4 represents an interface between the storage system 2 and the users N1, N2, N3, ...Nn, wanting to access such data, for example via a network 20 such as the internet. Such users are exemplary identified as:
- Referring/Satellite Hospital/practice which represents either the original institution which provided the data and those institutions authorized to handle the full data such as Government institutions or hospitals having the right to access the data of a particular patient for example for a follow-up exam of the same;
- Hospital user which represents the patient having the right to access his own data;
- Researchers, Medical imaging companies, Pharma companies, Health Organizations which are not authorized to access the full data, particularly those data having private patient information.

The parser 4 is typically a software unit which runs portions of software code for managing the access to data once the requester has been identified. If the requester is identified as authorized to accede the full data, the parser 4 allows the access to the original data, if not the access is granted to a modified version of the original data, namely the modified files 22'.

As the files archived in the repository 2 are typically Dicom files, a modified version of the original data may consist in files having the relevant Dicom tags related to patient personal data masked or cleared. Fig. 4 shows a partial list of Dicom tags which are cleared by the Free Dicom Anonymizer by WebPax (see http://www.webpax.com) and which could, in part or in full, be exemplary considered also in the present invention.

As various imaging devices place patient identification strings also in the form of pixels in image areas, the invention advantageously provides for the deletion of patient identification data from the images with appropriate masks to be superimposed on such pixels. As each modality has its own way of graphically presenting patient information on an image, the invention preferably provides for the identification of the vendor of the acquisition device to select the appropriate mask to be used for clearing patient information on each image.

It is, in fact, common for every imaging device to provide in the image raster also some pixel areas dedicated to the patient name, gender and birth date, however such information is generally positioned in different locations of the image which thus needs to be addressed appropriately with a priori knowledge of such locations as a function of the acquisition device.

The process of modification of data for allowing access to non-authorized users will be hereinafter referenced as a process of anonymization as privacy is the main issue of the present invention, however the skilled person would appreciate that also different way of modifying the original data can be considered so that different data can be transparently and automatically presented to different users according to the access rights they possess. For example it may happen that institutions authorized to access the full patient data are indeed forced to accede type of data different from the original data. Such modified data could simply consist in a part of the whole patient record. For example reports, comments or just the personal data of the physician making such reports or comments could be masked to avoid possible bias or prejudice in second diagnosis.

The modification of the data can be affected once the data have been transferred to the repository, for example in batch mode, or "on the fly" when there's an access request from an unauthorized recipient. In the former case each document is stored in the repository both in the original and in the modified form for a quicker access while in the latter case documents are stored only in the original format and the conversion is made when a request for an non-authorized access is pending, for example using memory buffers. Alternatively, each document is converted "on-the-fly", but, instead of being placed in a buffer, it is stored in the repository together with the original document for an immediate subsequent non-authorized access. Hence the process of modification of data can be part of the parser 4 and/or of a separate component, typically a software unit, which acts directly on the storage system 2 or on the incoming data during the upload process. In any case the result is a system which is capable to manage relevant medical information in the original form or in a modified form suitable to be shared among a potentially indefinite number of people.

The mechanism for managing the access to data is based on the identification of the receiver with reference to the owner of the information, namely the sending institution and/or the patient. In an embodiment of the present invention, the original institution which performs the examination, which is to be sent to the central repository 2, is identified with a code indicated in Fig. 3 as Clinical Institution Code 30 (CIC). Such code could be, for example, univocally assigned to the institution upon installation or registration by an identity provider 40 associated to, or part of, the system. It could also be any identification otherwise used to identify the institution. The patient undergoing an examination can be identified with patient data 31 (such as a patient code), called Personal Identification Code (PIC) in the embodiment of Fig. 3, to be furnished by the system, e.g. through the identity provider (40) or with his personal identification code furnished, for example, by the National Health Service. Any system of identification of the Clinical Institution or the Patient is equivalent for the purpose of the present invention as long as the related code is unique in the system. This could be achieved, for example, by adding a prefix and/or a suffix to an elsewhere furnished code.

In an embodiment, each examination made by institution CIC on the patient PIC is identified by a code, which is referenced in Fig. 3 as Exam Retrieval Code 37 (ERC), which can be, in principle, the only code to be used for handling a piece of healthcare information by the system. Indeed, a differential access to information by means of parser 4 requires for at least the identification of the acquisition institution 7 to allow the sender of information to have free access in browsing/downloading. This could also be possible, for example, by assigning to the acquisition institution 7 a range of possible ERCs which the parser recognizes as originating from such acquisition institution 7.

If full access to the patient is also to be allowed, the ERCs of the data stored in the repository should somehow retain the information on the patient to allow the parser to match the patient with his data. Such identification could be possible through the original acquisition institution 7 or an affiliate institution which keeps track of the identification of each patient with reference to an ERC in its internal database. In this case the patient can access its own record in full, for example by using a code and a password provided by the institution to the patient and recognized by the parser 4 as a means for allowing access to a particular exam without the need to identify the patient with his PIC.

In a preferred solution, the Exam Retrieval Code 37 (ERC) contains data directly related to the exam, identified in Fig. 3 as Exam Identification Code 32 (EIC), like date and time of acquisition and the type of imaging device used, but also to the CIC and the PIC. The ERC could be formed, for example, by simply placing the three codes side by side in any order or by using a particular codification which encapsulates the three codes in a single code. The function of generating the ERC is subtended by the module indicated with reference 5 in Fig. 3. The generated ERC is transmitted to the Dicom Storage system 2 via the link 501 together with the corresponding Dicom files (see link 201). If the institution has a local backup database 3, the ERC is also transmitted, together with the images, to such database as shown by the links 601 and 301 in Fig. 3. The locally stored images can be of any form, Dicom included, depending on the local storage/retrieval system which could in principle be any kind of PACS according to the prior art.

The storage system 2 can be of any known kind, but it is preferably a Web-compatible database which is part of a http server that can be interrogated from any location on earth, for example, using a standard Internet browser.

The parser 4 has the form of a search engine, for example based on the execution of CGI scripts for querying the database as it is well known in the world of Internet. The parser 4 represents the interface between the database 2 and the users N of the system. The parser 4 is mainly a software tool that authenticates a user, for example with a username and a password, to redirect his access to the correct data. The parser 4 may also execute a script that modifies the data located in the database if the user is not recognized as having the rights to access the full original data and such modified data are not stored in the database as seen above. Any kind of authentication is possible, for example using smart cards or software certificates like PKCS to improve security, as long it allows the parser 4 to identify the user with certainty. Such user may be the acquisition institution 7, a patient 6 or any other user 8.

If the acquisition institution 7 attempts to access some data via the link 401 in Fig. 3, the parser 4 verifies if the authorization code 42 such as the CIC of the institution matches the CIC codified in the ERC stored in the database 2 along with the data as seen above. In the affirmative case, the parser 4 allows full read/write access to such data, i.e. the institution is not only allowed to parse and download the data, but also to modify or replace them or simply append further information such as a second diagnosis or other images acquired with a different modality as it normally happens in PACS systems.

If the CIC codified in the ERC is not matched with the authorization code 42 such as the CIC of the institution attempting to access some records, the institution is not recognized as the owner of the requested information and the access is allowed only to the modified records (see box 7) unless the patient is present and furnishes his own authentication code 42 such as the PIC together with the CIC of the institution.

This may be the case of an institution performing a follow-up examination or a second diagnosis on a patient. Access to the full information of the patient is possible only if the patient gives his authorization. This access modality is represented in Fig. 3 by the other institution (other user) 8. In this case the institution has no rights to modify the original data (see link 701), but can append further information (see link 801) like other images or diagnostic reports that will become available to everyone in the original and the modified version as any other piece of information in the system.

If the patient attempts to access some data via the link 901, the parser 4 verifies if the PIC of the patient matches the PIC codified in the ERC stored in the database 2 along with the data. In the affirmative case, the parser 4 allows the patient to read/download his data. This access modality is represented by box 6 of Fig. 3.

Other type of access is considered as non-authorized in the sense that only a modified version of the data can be parsed and/or downloaded. This is represented by the other institution (other user) 8 and link 111 in Fig. 3. Such type of access is, in general, a read-only access. However it can be possible also to provide for some append capability to allow the users, for example, to post comments like in the Webpax service or any other forum or community social networks like Google Health (http://www.google.com/health). This is possible because these data are anonymized data that can be freely shared within the community. All data are shared, not only those provided by the patients, thanks to the advantageous way of automatically handling documents/images which represents the core of the invention. Furthermore these data come from original institutions which performed the examination and thus are intrinsic reliable. Access to anonymized data can, in principle, be granted to everyone, although some kind of control is preferably provided for example by authenticating the user wanting to access such data. This also allows for the set up of a system capable of handling access under payments of some fees. For example commercial institution could gain access upon payment of some moneys to be shared among the sending institutions as an incentive to provide best quality data for their distribution to the community.

According to an improvement, anonymized access, but also access to original data, can be logged to allow users to check which data are more frequently accessed. The data used in clinical trials, for example, can be certified by a system access control to allow the conclusion of scientific research to be reconstructed to improve the control of diagnostic or therapeutic conclusions reducing the occurrence of biased results.

The parser 4 can be coupled with a relational database system that allows the retrieval of information on the basis of a series of search elements, related, for example, to the type of diseases, to the therapeutic procedures, to the available characteristics of the patients, the multimodal completeness of available images and so on. In this way the retrieval of pertinent information is speeded-up.

In a further embodiment, the parser 4 can redirect the access through a series of tools that perform quantitative operation on the data. For example image quantification tools can analyze the image data to allow the access to the results of the automated quantification in place of the original images. A series of data may be required in statistical terms only, such that the user accedes the synthetic statistical results in place of the whole original data. Such a parser may potentially redirect the data for performing intensive calculation operations, like numerical simulations, to allow the access to the results of computation that are typically not available within single institutions. This computation power can be granted by the unlimited resourced distributed on the web, like the cloud computing, and allows to perform advanced studies at an unprecedented level.

Although the system according to the invention has been mainly described with reference to a repository which, for the sake of simplicity, has been sketched and referred mainly as a big database centrally located, it can be appreciated that data can also be equally distributed worldwide under the supervision of a central server, all without departing from the guiding principle of the invention disclosed above and claimed below.

## Claims

1. A medical data management method comprising the steps of:
o receiving medical data (22c) associated with a patient (6),
o receiving identification data (22b) associated with the medical data (22c) of said patient (6),
o storing said medical data (22c) and said identification data (22b) as a
medical file (22) in a storage system (2),
**characterized in** comprising the further steps of:
o modifying at least parts of said medical files (22) and generating at least one modified file (22') in response to a retrieval request from an unauthorized recipient (Ni), wherein such modification depends on an authorization code (42) associated with said unauthorized recipient (Ni), and
o supplying said at least one modified file (22') to said unauthorized recipient (Ni) and/or supplying the unmodified medical file (22) to an authorized recipient (6, 7).

2. The medical data management method according to claim 1, comprising the further step of providing an identity provider (40) for providing said identification data (22b) associated with said patient (6), an acquisition institution (7) and/or other identification data, such identification data (22b) being supplied at least partially to the storage system (2) by said acquisition institution (7) to identify said medical data (22c).

3. The medical data management method according to one of claims 1 or 2, comprising the further step of receiving acquisition data (22a) associated with the acquisition method for obtaining said medical data (22c) associated with said patient (6), wherein the acquisition institution (7) generates said acquisition data (22a) associated with the medical data (22c) of said patient (6) and supplies said medical file (22) to said storage system (2) via a parser (4).

4. The medical data management method according to one of the preceding claims, **characterized in that,**
said parser (4) modifies at least parts of said medical file (22) and generates a modified file (22') either in response to a retrieval request from an unauthorized recipient (Ni) which depends on authorization codes (42) associated with different unauthorized recipients (Ni) and/or in response to a modification program.

5. The medical data management method according to claim 4,
**characterized in that,**
said modification program generates at least one modified file (22') by using filtering, data mining, segmenting, classifying and/or standardizing said medical data (22c).

6. The medical data management method according to one of the preceding claims,
**characterized in that,**
said parser (4) generates a modified file (22') by categorizing and/or normizing said medical data (22c) associated with such acquisition data, thereby generating 2^{nd} level data wherein such parser comprises preferably calculation means for and/or normizing said medical data (22c).

7. The medical data management method according to claim 6,
**characterized in that,**
said parser (4) is normizing and/or categorizing said medical data (22c) by comparison with certain standards and/or certain categories.

8. The medical data management method according to one of the preceding claims, **characterized in that,**
said identification data (22b) comprises an exam retrieval code (37) which comprises an institution code (30) with information related to the acquisition institution (7), patient data (31) with information related to the patient (6) and/or an exam identification code (32) with information related to the examination modalities.

9. The medical data management method according to one of the preceding claims, **characterized in that,**
said authorization code (42) is compared with said identification data (22b) of the requested medical file (22) in order to determine those parts of said medical file (22) which have to be modified for generating said modified file (22').

10. The medical data management method according to one of the preceding claims, **characterized in that,**
said medical data (22c) is additionally stored in a modified, preferably anonymized version, and
said acquisition data (22a) and said modified medical data (22c) are supplied as said modified file (22') to any unauthorized recipient (Ni) in accordance with the authorization level of said unauthorized recipient (Ni).

11. A medical data management system (50) comprising,
a. a gateway (21) for receiving medical data (22c) associated with a patient (6), and identification data (22b) associated with the medical data (22c) of said patient (6), and
b. a storage system (2) for storing said medical data (22c) and said identification data (22b) as a medical file (22),
**characterized in that,**
c. a parser (4) modifies at least parts of said medical files (22) and generates at least one modified file (22') in response to a retrieval request from an unauthorized recipient (Ni), wherein such modification depends on an authorization code (42) associated with said unauthorized recipient (Ni), and
d. said parser (4) supplies said modified file (22') to said unauthorized recipient (Ni) or supplies the unmodified file (22) to an authorized recipient (6, 7) via said gateway (21).

12. The medical data management system according to claim 11,
wherein said acquisition institution (7) and/or said recipient (N) and said medical data management system (50) comprises a PACS and connecting means for interfacing such PACS with said storage systems (2) to transfer medical files (22, 22') with medical data (22c) including one or more imaging modalities.

13. The medical data management system according to claims 11 or 12,
wherein said medical files (22) and/or said modified files (22') are arranged in such storage system (2) in a hierarchical way to provide different permissions for accessing said medical files (22) and/or said modified files (22') as a function of the authorization code (42) associated with said unauthorized recipient (Ni)

14. A medical data management system according to claims 11 - 13,
wherein a recipient identified as said patient (6) has read-only permissions to access his own medical files (22) and wherein a recipient identified as said acquisition institution (7) has full read/write access to said medical files (22) and/or modified files (22').

15. A medical data management system according to claims 11 - 14,
wherein modified files (22') comprise digital medical images that are anonymized by clearing the pixels values containing such data on the image file.

16. A medical data management system according to claims 11 - 15,
wherein said parser (4) modifies several medical files (22) by performing calculation operations, like numerical simulations, and generates a modified file (22') which includes data of said several medical files (22), such as statistical data.

17. A software module for a medical data management system according to claims 11 - 15 or for a medical data management method according to one of claims 1 - 10, comprising means for reading said medical files (22) from said storage system (2), for modifying said medical files (22), for comparing authorization codes (42) with identification codes (22b) and for supplying said at least one modified file (22') to said unauthorized recipient (Ni) or supplying the unmodified medical file (22) to an authorized recipient (6, 7).
